# EUROPEAN PATENT APPLICATION

(11) **EP 1 782 805 A1**
(43) Date of publication of application: **09.05.2007**
(21) Application number: 05768493.8
(22) Date of filing: 04.08.2005
(51) Int. Cl.: A61K 31/191, A61K 31/365, A61P 1/14, A61P 3/14, A61P 19/10, A61P 43/00, C07D 307/33, A23L 1/304, A23L 1/30

(54) **COMPOSITION FOR ACCELERATING CALCIUM ABSORPTION**

(30) Priority: 05.08.2004 JP 2004229792; 05.08.2004 JP 2004229793
(71) Applicant: Godo Shusei Co., Ltd., Tokyo, 1048162 (JP)
(72) Inventor: SANAI, Kazuko, Godo Shusei Co., Ltd., Matsudo-shi, Chiba, 271-0064 (JP); TANAKA, Yuko, Godo Shusei Co., Ltd., Matsudo-shi, Chiba, 271-0064 (JP); NEGISHI, Shigenori, Godo Shusei Co., Ltd., Matsudo-shi, Chiba 271-0064 (JP); SERI, Kenji, 3400004 (JP); SASAKI, Hironori Godo Shusei Co., Ltd., Matsudo-shi,Chiba, 271-0064 (JP)
(74) Representative: Hartz, Nikolai
(86) International application number: PCT/JP2005/014312
(87) International publication number: WO 2006/013929

(57) **Abstract**

The present invention relates to a composition for accelerating calcium absorption containing a poorly absorbable L-form aldonic acid, a salt thereof, or a poorly absorbable L-form aldonolactone. The invention also relates to a food or beverage containing L-arabonic acid, a salt thereof, or L-arabonolactone.

The invention also relates to a process for preparing L-arabono-γ-lactone crystals, **characterized by** dissolving L-arabonic acid in an organic solvent and crystallizing L-arabono-γ-lactone from the solution.

According to the invention, a food or pharmaceutical product effective in the prevention or treatment of osteoporosis can be provided.

## Description

### Technical Field

The present invention relates to a composition for accelerating calcium absorption, and a composition for prevention and treatment of osteoporosis.

### Background Art

Osteoporosis is defined to be a disease in which bone mineral content such as bone calcium content is decreased, thus inducing a decrease in the bone strength, and therefore resulting in an increased risk of bone fracture. It is assumed that the number of osteoporosis patients has been reached 10 billion along with the rapidly increase in population ratio of elderly people in recent years. While most of the patients are aged women, it has been reported that young people also suffer from lowered bone mineral content. Internal factors such as aging or menopause, and external factors such as insufficient calcium ingestion or insufficient exercise are known as the causes of such lowering in the bone mineral content. A report on the national nutrition survey issued by the Ministry of Health, Labour and Welfare in Japan revealed that the level of calcium ingestion is below the nutritional requirement.

For the purpose of supplementing the insufficient calcium ingestion, calcium supplements have been used. For example, calcium carbonate, calcium phosphate, bone powder, eggshell, calcium lactate, calcium D-gluconate, calcium L-ascorbate (Patent Document 1), calcium L-threonate (Patent Document 2), and the like are known. However, such calcium supplements have a problem that their effects are not sufficient because the capability for absorbing calcium from the digestive tract decreases due to aging or menopause. Furthermore, calcium L-ascorbate used in Patent Document 1 has a safety problem of inducing urolithiasis upon excessive ingestion because its L-ascorbic acid moiety is also absorbed. Moreover, Non-Patent Document 1 discloses that when L-threonic acid, which is produced by oxidative degradation of L-ascorbic acid, is orally administered to guinea pigs repeatedly for 4 to 28 days, the ascorbic acid content in the adrenal gland or testis is significantly decreased, thus calcium L-threonate used in Patent Document 2 also has a safety problem. In order to solve such problems, there is a need for a material which is capable of accelerating absorption of the calcium component contained in food products from the digestive tract as well as having improved safety.

As a material which accelerates absorption of calcium in the digestive tract, galacto-oligosaccharides (Patent Document 3), fructo-oligosaccharides (Patent Document 4), α-glucosidase inhibitors (Patent Document 5) and the like are known, however their effects have been insufficient. One reason for such insufficiency is that the main site of action of these materials is located in the posterior part of the digestive tract such as large intestine. That is, after the indigestible oligosaccharides used in Patent Documents 3 or 4 reach the large intestine, they shift the pH in the large intestine to the acidic side under the action of intestinal bacterial flora, thereby accelerating calcium absorption in the large intestine region. The material used in Patent Document 5 suppresses digestion of carbohydrates in foods so that the undigested carbohydrates reach the large intestine and accelerate calcium absorption with the same mechanism as Patent Documents 3 and 4. However, since an important absorptive site for calcium is located in the anterior part of the small intestine, the amount of calcium absorbed from the large intestine region by the action of these materials is inevitably small.
[Patent Document 1] Japanese Patent Application Laid-open No. H9-157174
[Patent Document 2] Japanese Patent No. 3315947
[Patent Document 3] Japanese Patent No. 3179090
[Patent Document 4] Japanese Patent Application Laid-open No. H7-252156
[Patent Document 5] Japanese Patent No. 2531273
[Patent Document 6] Japanese Patent Application Laid-open No. 2001-327298
[Patent Document 7] Japanese Patent Application Laid-open No. H11-32788
[Non-Patent Document 1] Thomas M. and Hughes R., Food and Chemical Toxicology, Vol. 21, pp. 449-452 (1983).

### Disclosure of the Invention

### Problems to be Solved by the Invention

The object of the present invention is to provide a composition which accelerates absorption of calcium component such as food-derived calcium, as well as having improved safety.

### Means for Solving the Problems

We have thought that a material which accelerates calcium absorption by exerting its action in the small intestine region, which is a predominant absorptive site for calcium, would give dramatic effects over conventional materials. Then, we have found that poorly absorbable L-form aldonic acids, or salts or lactones thereof, whose function have been heretofore unknown, exerts a prominent action of accelerating calcium absorption in the small intestine, a predominant absorptive site for calcium, and useful for prevention and treatment of osteoporosis. Furthermore, we also have found that, among the aforementioned substances, calcium L-arabonate is useful as a calcium supplement and is useful for prevention and treatment of osteoporosis.
As a result of further research, we also have found that, among the L-form aldonic acids, L-arabonic acid, a salt or lactone thereof not only has a calcium absorption accelerating action, but also is useful as an acidic material, a pH buffer or a salt replacement for a variety of food products.
In addition, L-arabonic acid has not been crystallized and has had problems in its miscibility for using in foods or pharmaceuticals. However, we found that crystals of L-arabono-γ-lactone can be readily obtained when a solution obtained by dissolving L-arabonic acid in an organic solvent is subjected to crystallization to precede dehydration reaction and crystallization simultaneously.

Thus, the present invention is to provide a composition for accelerating calcium absorption containing a poorly absorbable L-form aldonic acid, a salt thereof, or a poorly absorbable L-form aldonolactone, and a composition for prevention and treatment of osteoporosis.
The present invention is also to provide a calcium supplementary food product containing calcium L-arabonate.

Further, the present invention is to provide a dietary product containing L-arabonic acid, a salt thereof, or L-arabonolactone.

Moreover, the present invention is to provide a process for preparing L-arabono-γ-lactone crystals, characterized by dissolving L-arabonic acid in an organic solvent, and crystallizing L-arabono-γ-lactone from the resulting solution.

### Effects of the Invention

The poorly absorbable L-form aldonic acids, salts, or lactones thereof disclosed in the present invention have a prominent action of accelerating calcium absorption in the small intestine, which is a predominant calcium absorption site. Ingestion of a food product to which the composition disclosed in the present invention has been previously added, or ingestion of the composition of the present invention together with calcium-containing food products such as dairy products, fish or meat, improves the absorption rate of calcium contained in the food products but poorly absorbed. Consequently, the lowered bone mineral content in osteoporosis patients is increased, thus the risk of bone fracture being decreased.

In addition, the effects of the present invention are particularly remarkable in aged people with decreased capability for calcium absorption, wherein the lowered bone mineral content in osteoporosis patients is increased, thus the risk of bone fracture being decreased.

L-arabonic acid has an action of accelerating the absorption of dietary calcium as well as an excellent pH buffering capacity and a function for replacing salt. Since L-arabonic acid does not have a strong taste, it is useful as a food additive, and can be applied to a wide range of dietary products.

Moreover, according to the present invention, an L-arabono-γ-lactone crystal can be readily prepared. Therefore, the convenience in storage and management is significantly enhanced because the crystal can be metered precisely and its applications in the solid form become expanded, and because the crystals can be used as L-arabonic acid when dissolved in water.

### Brief Description of the Drawings

Fig. 1 is a graph showing the action of L-arabonic acid on dietary calcium absorption in Example 1.
Fig. 2 is a graph showing the blood calcium level after the administration of calcium L-arabonate in Example 5.
Fig. 3 is a graph showing the neutralization curve upon dropwise addition of sodium hydroxide into an aqueous solution of the preparation of Example 9 of the present invention.
Fig. 4 is a graph showing the neutralization curve upon dropwise addition of sodium hydroxide into aqueous solutions of L-arabono-γ-lactone and D-glucono-δ-lactone in Example 9.
Fig. 5 is a diagram illustrating the structure obtained by X-ray crystal diffraction of L-arabono-γ-lactone.

### Best Mode for Carrying Out the Invention

The L-form aldonic acids, salts or lactones thereof used in the present invention can be prepared by any known method such as a method of making a microorganism which is capable of aldose oxidation or an aldose oxidase enzyme act on an L-form aldose, or a method of oxidizing aldose by a chemical oxidation reaction. For example, Patent Document 6 describes a process for preparing an acidic carbohydrate, characterized by making glucose oxidase act on arabinose or xylose, and a process for preparing an acidic sugar-calcium salt, characterized by adding calcium upon the action of glucose oxidase. Patent Document 7 discloses a method for preparing an acidic carbohydrate and/or an acidic carbohydrate-related compound, characterized by making the action of glucose oxidase act on a carbohydrate excluding glucose. However, the physiological function of the products, arabonic acids or xylonic acids and compounds related thereto, has not been reported in any document including Patent Document 6 and 7. For the present invention, L-arabonic acid, a salt thereof or L-arabonolactone is particularly preferred.

Examples of the L-form aldose include L-glyceraldehyde, L-threose, L-erythrose, L-lyxose, L-xylose, L-arabinose, L-ribose, L-talose, L-galactose, L-idose, L-gulose, L-mannose, L-glucose, L-altrose, L-allose, L-glycero-D-manno-heptose and the like. L-form deoxyaldoses such as L-fucose, L-rhamnose and the like are also be included in the starting material. The resulting aldonic acid usually has its lactone form existing in equilibrium.

An example of the process for preparation includes inoculating Acinetobacter baumannii, a microorganism which is capable of aldose oxidation, into a liquid culture medium containing L-arabinose, and culturing the medium with aeration and agitation. During the course of culturing, L-arabinose in the culture medium is decreased and L-arabonic acid is produced. If calcium carbonate is previously added to the medium, pH of the culture medium is stabilized, and a calcium salt of the produced L-arabonic acid can be obtained. Once L-arabinose in the culture medium has been all consumed, the medium can be filtered to remove solid components such as the fungus body, then the resulting solution can be purified by any known methods such as processing with an ion exchange resin column, and L-arabonic acid of high purity can be obtained. Alkali metal salts of L-arabonic acid can be prepared by known methods.

In the case of using another L-form aldose or L-form deoxyaldose as the starting material, the corresponding L-form aldonic acid, a salt or lactone thereof can be prepared in the same manner.

The salt of L-form aldonic acid of the present invention includes alkali metal salts such as sodium salt, potassium salt and the like; alkaline earth metal salts such as calcium salt, magnesium salt and the like; and metal salts such as copper salt, iron salt, zinc salt and the like. The L-form aldonic acid, a salt or lactone thereof can be used alone or as a composition with carriers such as excipient, in the form of powder, granule, solution or the like. The composition of the invention includes pharmaceuticals in the form of powder, granule, tablet, capsule, syrup and the like, food additives, functional food products and ordinary food products, all of which contain an L-form aldonic acid, a salt or lactone thereof as an active ingredient. The daily intake/dose of the L-form aldonic acid, a salt or lactone thereof of the invention for an adult is 0.5 g to 30 g, preferably 1 g to 5 g.

In the case of using L-arabonic acid, a salt thereof or L-arabonolactone as a food additive, other organic acids, organic acid salts, additives such as anticaking agent, excipient, antioxidant, common salt, flavor, various esters, sugars, colorant and the like may be added.

The other organic acids and salts thereof used in the present invention are edible acids or salts thereof that can be used as food additives, and examples of the organic acids include acetic acid, lactic acid, citric acid, malic acid, tartaric acid, fumaric acid, adipic acid, gluconic acid, and mixtures thereof, while examples of the organic acid salts include sodium salts, potassium salts, calcium salts thereof, and mixtures thereof.

The food additive of the present invention is prepared for use by formulating L-arabonic acid, a salt thereof or L-arabonolactone directly, or appropriately in combination with various anticaking agents, excipients or media, into a powder, a granule, a tablet, a liquid such as an aqueous solution, according to a standard method.

Examples of the food products that can be used according to the present invention include noodles such as boiled noodles, buckwheat noodles, wheat noodles, spaghetti and the like; articles of taste such as pickles, delicacies and the like; prepared foods such as boiled sweet potatoes, boiled red beans and the like; vegetables such as cut vegetables and the like; cooked rice, processed rice cake products, frozen fish, processed fish products such as fish sausages and the like, processed meat products such as ham, sausages and the like, fried food products such as croquettes, cutlets, fries and the like, soybean paste, sauces, fruit juice beverages, dairy products, processed soybean products, confectionaries such as candies, refreshing drinks such as sports drinks, and dough of bakery products such as breads, cakes, cookies and the like.

The method of adding L-arabonic acid, a salt thereof or L-arabonolactone to the food products of the present invention is not particularly limited, but rather conducted by any means including mixing, spreading, or spraying into the food products to be treated or materials thereof during production, processing or cooking of the food products. For example, L-arabonic acid, a salt thereof or L-arabonolactone can be, without limitation, directly mixed with the raw materials, added to a processing liquid such as cooking liquid, or added to a liquid for spraying or immersion. The L-arabonic acid, a salt thereof or L-arabonolactone may be mixed with other components previously to prepare a food additive, or other components can be mixed separately as necessary.

The amount of L-arabonic acid, a salt thereof, or L-arabonolactone to be added to a food product according to the present invention may be appropriately increased or decreased, depending on the type, taste, method of preparation (processing temperature, processing time, etc.), or the purpose of use (improvement of storability, prevention of oxidation, prevention of discoloration, etc) of the food product. The effective amount for addition is preferably determined not only by preparation test but also sensory evaluation and the like. The amount for addition is preferably, for example, 0.00001 to 10% by weight.

According to the invention, L-arabono-γ-lactone crystals can be obtained by dissolving L-arabonic acid in an organic solvent, and crystallizing L-arabono-γ-lactone from the resulting solution.

With regard to the L-arabonic acid to be used as a raw material in the method of the invention, an L-arabonic acid solution may be prepared by loading a commercially available reagent of calcium L-arabonate to a cation exchange resin, or an L-arabonic acid solution which is obtained by converting L-arabinose into L-arabonic acid in culture medium under the action of a microorganism or an enzyme which is capable of aldose oxidation, and purifying the L-arabonic acid using an ion exchange resin or the like, can be used. Among these, it is preferable to use the L-arabonic acid obtained by making a microorganism or an enzyme which is capable of aldose oxidation act on the L-arabinose.

According to the present invention, L-arabonic acid is dissolved in an organic solvent. The organic solvent that can be used is preferably acetone, methanol, ethanol, acetonitrile, or a mixture thereof. It is preferable to dissolve L-arabonic acid in such organic solvents at 20 to 60°C.

When the resulting solution is concentrated, a dehydration reaction of L-arabonic acid occurs, and L-arabono-γ-lactone is crystallized out. Furthermore, in the case of using an aqueous L-arabonic acid solution as L-arabonic acid, an organic solvent can be added to the solution to concentrate the solution repeatedly, to distill off the water and to dissolve L-arabonic acid in the organic solvent. Crystals of L-arabono-γ-lactone are obtained by subjecting the concentrated solution to crystallization at a low temperature, or by adding seed crystals to promote crystallization. Herein, it is preferable to perform the concentration at a reduced pressure.

L-arabono-γ-lactone crystals can also be obtained by adding a poor solvent such as ethyl acetate, propanol or the like to the resulting solution, and subjecting the solution to crystallization. The solvent added as the poor solvent is not limited to the ethyl acetate or propanol as described above.

The obtained L-arabono-γ-lactone crystals can be easily isolated by solid-liquid separating means such as filtration. The crystals are stable under normal temperature and pressure, and thus have good handleability.

### EXAMPLES

Hereinafter, the present invention will be described in detail, but the invention is not intended to be limited to the following Examples.

### Example 1

### Dietary calcium absorption accelerating effect of L-arabonic acid

For the experiment, 11-weeks old male ICR mice were used after overnight fasting. During the test period, the mice were given deionized water only. The mice were divided into 8 groups (Groups I to VIII, 5 animals in each group), and all of the groups were orally administered with 5 mmol/kg of calcium carbonate, which corresponds to a dose of 200 mg of calcium/kg. Group I was administered with calcium carbonate only, Groups II to V were orally administered with calcium carbonate and L-arabonic acid simultaneously, and Groups VI to VIII were orally administered with calcium carbonate and D-gluconic acid simultaneously. The doses of L-arabonic acid administered in combination were 1.25 mmol/kg (Group II), 2.5 mmol/kg (Group III), 5.0 mmol/kg (Group IV), and 10.0 mmol/kg (Group V), and the doses of D-gluconic acid were 2.5 mmol/kg (Group VI), 5.0 mmol/kg (Group VII), and 10.0 mmol/kg (Group VIII).

To examine the changes in the blood calcium level as an index for the calcium absorption accelerating effect, 10 µL blood samples were collected from the orbital vein of each mice using a capillary tube, before and 15 minutes, 30 minutes, 60 minutes, 90 minutes, 2 hours and 4 hours after the administration of the test substance. The blood calcium level was quantified by a chelate colorimetric method (ortho-cresolphthalein complexone (OCPC) method). The obtained data were analyzed by a paired t-test, and a risk ratio of 5% or less was considered as significant difference.

As a result, Group I in which calcium carbonate was administered alone did not show any increase in the blood calcium level up to 4 hours after the administration, while Groups II to V in which calcium carbonate and L-arabonic acid were administered in combination were observed to have dose-dependent and significant increases in the blood calcium level. Meanwhile, Groups VI to VIII in which calcium carbonate and D-gluconic acid were administered in combination were not observed to have significant increases in the blood calcium level.

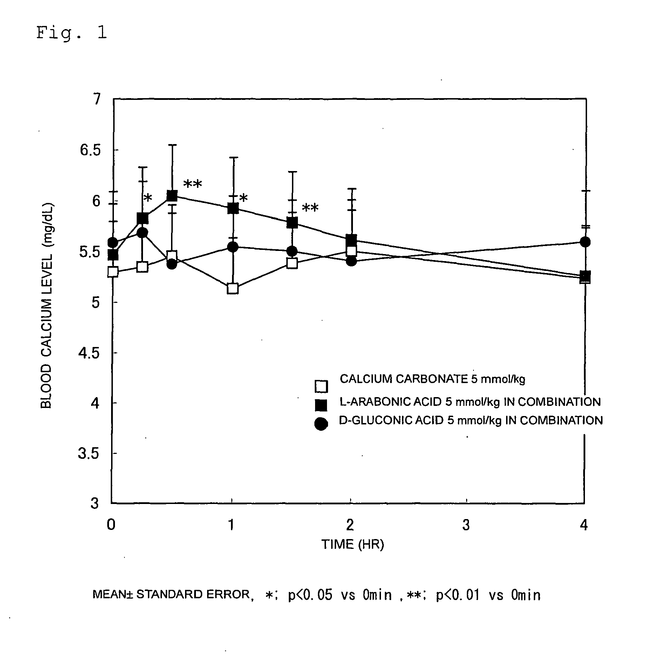

Fig. 1 shows the changes in the blood calcium level of Group I in which calcium carbonate was administered alone, Group IV in which calcium carbonate and 5 mmol/kg of L-arabonic acid were administered simultaneously, and Group VII in which calcium carbonate and 5 mmol/kg of D-gluconic acid were administered simultaneously. It is obvious that calcium absorption was accelerated by L-arabonic acid, and the effect was rapidly acting and continuous.

### Example 2

### Dietary calcium absorption accelerating effect of sodium L-arabonate

Eight-week old male ICR mice were used and fed calcium-deficient diet and deionized water for one week. The mice were fasted overnight before the test, and were given deionized water only during the test period. The mice were divided into two groups, and Group I was administered with 5 mmol/kg of calcium carbonate alone, while Group II was orally administered with 5 mmol/kg of calcium carbonate and 5 mmol/kg of sodium L-arabonate simultaneously. The measurement of the blood calcium level was performed in the same manner as in Example 1. The obtained data were analyzed by a paired t-test, and a risk ratio of 5% or less was considered as significant difference.

As a result, as shown in Table 1, Group I in which calcium carbonate was administered alone did not show any increase in the blood calcium level up to 4 hours after the administration, while Group II in which calcium carbonate and sodium L-arabonate were administered in combination were observed to have a significant increase in the blood calcium level concentration in the blood.

**[Table 1]**

| Group | Effect of sodium L-arabonate on dietary calcium absorption | | |
|---|---|---|---|
| | Ca level in blood (mg/dL) | | |
| | Time after administration | | |
| | 0 min | 2 hrs | 4 hrs |
| I | 4.67±0.297 | 4.58±0.143 | 4.82±0.189 |
| II | 4.30±0.121 | 5.59±0.232** | 5.62±0.212** |

| | | | |
|---|---|---|---|
| Mean ± standard error, n=4, **; p<0.01 vs 0 min | | | |

### Example 3

### Kinetics of L-arabonic acid in the digestive tract of rat

For the experiment, 7 to 11-weeks old male Wistar rats were used after overnight fasting. After orally administering 5 mmol/kg of L-arabonic acid, the animals were entered into a metabolic cage, and were sacrificed after 2 hours, after 6 hours, and after 24 hours, to remove the digestive tracts. The contents in each site of the digestive tract were recovered respectively, and the amount of unaltered L-arabonic acid was quantified by the HPLC method. The entire amounts of urine and feces up to the respective time points were recovered, and the amounts of L-aranobic acid were quantified in the same manner. During the test period, the animals were given deionized water only.

The results are shown in Table 2. It was found that from 2 to 6 hours after the administration, most of L-arabonic acid was hardly absorbed in the small intestine and transferred to the cecum and colon without being altered. About 30% of the total amount of administration was remained in the cecum and colon after 24 hours of the administration, and L-arabonic acid was also detected from the excreted feces, suggesting that L-arabonic acid is poorly assimilated by intestinal bacteria. Unaltered L-arabonic acid was also detected from the urine, even in small amount, which indicating that a part of the L-arabonic acid reaching the cecum or colon was absorbed and excreted through the urine without being used in the body. Throughout the test period, the level of L-arabonic acid in the blood was below the detection limit.

**[Table 2]**

| Residual ratio of L-arabonic acid in digestive tract after oral administration | | | |
|---|---|---|---|
| Site in digestive tract | Residual ratio after administration (%) | | |
| | After 2 hrs | After 6 hrs | After 24 hrs |
| Stomach | 0 | 0 | 0 |
| Small intestine | 23.0±3.71 | 3.3±2.50 | 0.8±0.20 |
| Cecum | 47.7±3.39 | 60.6±3.26 | 25.9±7.81 |
| Colon | 0.1±0.19 | 7.4±2.35 | 1.9±0.37 |

| | | | |
|---|---|---|---|
| Mean ± standard error, n=4 | | | |

The residual ratio in the digestive tract of a salt of L-arabonic acid (calcium salt) after administration was also examined, and as a result, it was found that the arabonic acid moiety was hardly absorbed in the small intestine and transferred to the cecum and colon without being altered.
For the lactone of L-arabonic acid (L-arabono-γ-lactone), the measurement was also performed with a dietary calcium absorption accelerating composition in the same manner as in Example 1. As a result, the lactone of L-arabonic acid yielded the same effects as those of L-arabonic acid.

### Example 4

### Safety test for L-arabonic acid

As a result of a single oral test using mice, it was confirmed that the LD₅₀ value of L-arabonic acid was about 10 g/kg, and thus L-arabonic acid was a material having high safety.

### Example 5

### Blood Calcium level after administration of calcium L-arabonic acid

For the experiment, 11-weeks old male ICR mice were used after overnight fasting. During the test period, the mice were given deionized water only. The mice were divided into four groups (Groups I to IV, 5 animals in each group), and the test substance was orally administered as a solution in deionized water. The comparative group (Group I) was orally administered with 5 mmol/kg of calcium carbonate. The groups administered with calcium L-arabonate (Groups II to IV) were orally administered with 1.25 mmol/kg (Group II), 2.5 mmol/kg (Group III) and 5.0 mmol/kg (Group IV) of calcium L-arabonate, respectively. Ten microlitters blood samples were collected from the orbital vein of each mice using a capillary tube, before and 15 minutes, 30 minutes, 60 minutes, 90 minutes, 2 hours and 4 hours after the administration of the test substance. The blood calcium level was quantified by a chelating colorimetric method (ortho-cresolphthalein complexone (OCPC) method). The obtained data were analyzed by a paired t-test, and a risk ratio of 5% or less was considered as significant difference.

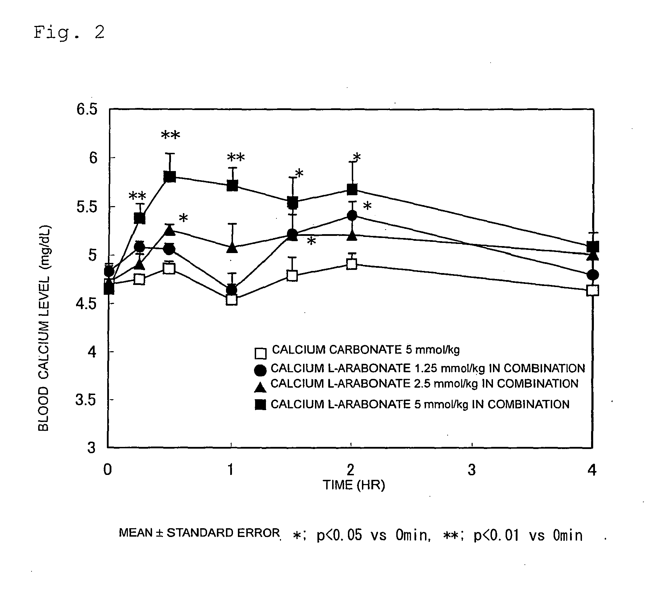

As a result, as shown in Fig. 2, the comparative group (Group I) in which 5 mmol/kg of calcium carbonate was administered did not show any increase in the blood calcium level up to 4 hours after the administration, while the groups administered with calcium L-arabonate (Groups II to IV) were observed to have dose-dependent increases in the blood calcium level. The increase in the blood calcium level due to the administration of calcium L-arabonate was rapidly acting and continuous. These results show that calcium L-arabonate is an excellent readily-absorbable calcium compound.

### Example 6

### Kinetics of calcium L-arabonate in the digestive tract of rat

For the experiment, 9 to 12-weeks old male Wistar rats were used after overnight fasting. After orally administering 2.5 mmol/kg of calcium L-arabonate, the animals were entered into a metabolic cage, and were sacrificed after 2 hours, after 6 hours, and after 24 hours, to remove the digestive tracts. The contents in each site of the digestive tract were recovered, respectively, and the amount of unaltered L-arabonic acid was quantified by the HPLC method. During the test period, the animals were given deionized water only.

The results are shown in Table 3. It was found that most of L-arabonic acid was hardly absorbed in the small intestine and transferred to the cecum and the colon without being altered from 2 to 6 hours after the administration,. About 23% of the total amount of administration was remained in the cecum and the colon after 24 hours of the administration, suggesting that L-arabonic acid is poorly assimilated by the intestinal bacteria.

**[Table 3]**

| Residual ratio of L-arabonic acid in digestive tract after oral administration of calcium L-arabonate | | | |
|---|---|---|---|
| Sites in the digestive tract | Residual ratio after administration (%) | | |
| | After 2 hrs | After 6 hrs | After 24 hrs |
| Stomach | 3.30±1.34 | 0.1±0.06 | 0 |
| Small intestine | 22.6±4.85 | 2.2±1.08 | 0.2±0.08 |
| Cecum | 34.4±5.81 | 57.8±2.27 | 19.0±15.67 |
| Colon | 1.9±0.76 | 3.9±1.30 | 2.5±0.95 |

| | | | |
|---|---|---|---|
| Mean ± standard error, dose of calcium L-arabonate: 2.5 mmol/kg, n=4 | | | |

While it was discovered in Example 5 that the calcium moiety of calcium L-arabonate used in the invention was extremely readily-absorbable, it was shown in Example 6 that the L-arabonic acid moiety was extremely poorly absorbed in the digestive tract. The in vivo kinetics of calcium L-arabonate is significantly different from those of calcium L-ascorbate and calcium L-threonate, which indicates that calcium L-arabonate used in the invention is superior to conventional substances in terms of safety. In a single oral administration test in mice, it was confirmed that the LD₅₀ value of calcium L-arabonate was 7.4 g/kg or more, and thus calcium L-arabonate was a substance having high safety.'

### Example 7

### Effects of calcium L-arabonate on experimental osteoporosis induced by calcium-deficient diet

Six-week old female ICR mice were divided into five groups (Groups I to V, 10 animals in each group), and fed calcium-deficient feed (calcium carbonate-free AIN 93M, Oriental Yeast Co., Ltd.) for 4 weeks to induce osteoporosis condition. Calcium L-arabonate was dissolved in water and orally administered daily in a dose of 1.25 mmol/kg (Group II) or 2.5 mmol/kg (Group III). The control group for pathological condition (Group I) was administered with water in the same manner. As a comparative group, a group administered with calcium D-gluconate (dose 2.5 mmol/kg (Group IV)) was provided. A normal control group (Group V) was separately provided and was given the normal feed (AIN 93M, Oriental Yeast Co., Ltd.). After administering the test substance on the second week and fourth week of initiation of the test, the animals were transferred to a metabolic cage, and feces were collected for 24 hours. The amount of calcium and phosphorus excreted in the feces were measured, and the absorption rates of ingested calcium and phosphorous in the digestive tract were determined. After completion of the test period, the animals of the respective groups were sacrificed to remove both femurs and tibiae. The femurs and tibiae were removed muscles etc., then immersed in 70% ethanol overnight, and dried at 60°C for 24 hours to be used as samples for analysis. The measurement items were dry weight, ash content, calcium content, and phosphorus content. The ash content was measured by an ashing method at 550°C for 18 hours, the calcium content was measured by an OCPC method (Calcium C-Test Wako, Wako Pure Chemical Industries, Ltd.), and the phosphorus content was measured by a p-methylaminophenol reduction method (Phospho C-Test Wako, Wako Pure Chemical Industries, Ltd.). The obtained data were analyzed by ANOVA and the Dunnett's method, and a risk ratio of 5% or less was considered as significant difference.

As a result, the absorption rates of calcium in the digestive tract on the second week and fourth week were 74.2% and 59.8%, respectively, for the calcium D-gluconate administered group (Group IV, comparative group), while the absorption rates were as high as 87.9% and 80.1%, respectively, for the calcium L-arabonate administered group (Group III). The absorption rates of calcium in the digestive tract for the low-dose calcium L-arabonate administered group (Group II) were even higher in values, and were 93.1% and 99.3%, respectively. The calcium L-arabonate administered groups (Groups II and III) also showed a tendency for higher absorption rates of phosphorus compared with the comparative group.

The weight, ash content, calcium content and phosphorus content of femur and tibia of the mice are shown in Table 4. The weight, ash content, calcium content and phosphorus content of femur and tibia of the group for pathological condition control (Group I) were all significantly reduced compared with those of the normal control group (Group V), exhibiting a osteoporosis condition. The groups administered with calcium L-arabonate (Groups II and III) were observed to be ameliorated in all of the above indices in dose-dependent manner, and the efficacy was superior to those of the calcium D-gluconate administered group (Group IV, the comparative group).

**[Table 4]**

| Effects of calcium L-arabonate on experimental osteoporosis induced by calcium-deficient diet | | | | | | | |
|---|---|---|---|---|---|---|---|
| | | Substance administered | Dose mmol/ kg | Weight mg | Ash content mg | Ca content mg | P content mg |
| Femur | Group I | Water | | 42±1.3⁺⁺ | 22±0.7⁺⁺ | 7.9±0.27⁺⁺ | 4.2±0.13⁺⁺ |
| | Group II | Calcium L-arabonate | 1.25 | 44±1.0 | 24±0.7 | 8.8±0.22 | 4.5±0.09 |
| | Group III | Calcium L-arabonate | 2.5 | 49±1.1** | 27±0.5** | 9.5±0.13** | 5.1±0.05** |
| | Group IV | Calcium D-gluconate | 2.5 | 46±2.1 | 25±1.3* | 8.7±0.42 | 4.7±0.21* |
| | Group V | Normal control group | | 52±1.5 | 30±1.1 | 10.9±0.38 | 5.5±0.20 |
| Tibia | Group | Water | | 42±1.1⁺⁺ | 21±0.8⁺⁺ | 7.6±0.17⁺⁺ | 3.9±0.08⁺⁺ |
| | Group II | Calcium L-arabonate | 1.25 | 43±1.6 | 23±0.8 | 8.1±0.28 | 4.2±0.14 |
| | Group III | Calcium L-arabonate | 2.5 | 46±1.2 | 24±0.5** | 9.1±0.22** | 4.6±0.05** |
| | Group IV | Calcium D-gluconate | 2.5 | 44±1.1 | 23±1.2* | 8.3±0.34 | 4.3±0.16 |
| | Group V | Normal control group | | 48±1.7 | 27±1.0 | 9.7±0.32 | 4.9±0.14 |

| | | | | | | | |
|---|---|---|---|---|---|---|---|
| Mean ± standard error; ⁺⁺, p<0.01 vs Group V; *, p<0.05 vs Group I; **, p<0.01 vs Group I | | | | | | | |

### Example 8

### Effects of calcium L-arabonate on experimental osteoporosis due to combination of ovary excision and calcium-deficient diet

Six-weeks old female ICR mice excised their ovaries were divided into 5 groups (Groups I to V, 10 animals in each group), and fed calcium-deficient feed (calcium carbonate-free AIN 93M, Oriental Yeast Co., Ltd.) for 4 weeks to induce osteoporosis condition. Calcium L-arabonate was dissolved in water, and orally administered daily in a dose of 1.25 mmol/kg (Group II) or 2.5 mmol/kg (Group III). The control group for pathological condition (Group I) was administered with water in the same manner. As a comparative group, a group administered with calcium D-gluconate (dose 2.5 mmol/kg (Group IV)) was provided. Also, a group excised ovaries but given with normal feed (AIN 93M, Oriental Yeast Co., Ltd.) (Group V) was separately provided. The measurement of the absorption rate of calcium in the digestive tract, and the analysis of femur and tibia were performed in the same manner as in Example 7.

As a result, the absorption rates of calcium in the digestive tract on the second and fourth week were 61.1% and 45.1%, respectively, for the calcium D-gluconate administered group (Group IV, comparative group), while the absorption rates were as high as 74.4% and 57.7%, respectively, for the calcium L-arabonate administered group (Group III). The absorption rates of calcium in the digestive tract for the low-dose calcium L-arabonate administered group (Group II) were even higher in values, and were 85.7% and 81.8%, respectively.

**[Table 5]**

| Effects of calcium L-arabonate on experimental osteoporosis induced by combination of ovary excision and calcium-deficient diet | | | | | | | |
|---|---|---|---|---|---|---|---|
| | | Substance administered | Dose | Weight | Ash content | Ca content | P content |
| | | | mmol/ kg | mg | mg | mg | mg |
| Femur | Group I | Water | | 43±1.2⁺⁺ | 23±0.6⁺⁺ | 7.6±0.12⁺⁺ | 4.2±0.06⁺⁺ |
| | Group II | Calcium L-arabonate | 1.25 | 45±0.9 | 25±0.4* | 8.8±0.18** | 4.7±0.07** |
| | Group III | Calcium L-arabonate | 2.5 | 50±0.9** | 28±0.8** | 10.2±0.24** | 5.3±0.11** |
| | Group IV | Calcium D-gluconate | 2.5 | 50±1.2** | 27±0.4** | 9.4±0.18** | 4. 8±0.14** |
| | Group V | Normal diet group | | 51±1.4 | 29±0.8 | 10.3±0.33 | 5.3±0.14 |
| Tibia | Group | Water | | 40±0.8⁺⁺ | 21±0.3⁺⁺ | 7.3±0.15⁺⁺ | 3.8±0.04⁺⁺ |
| | Group II | Calcium L-arabonate | 1.25 | 42±0.8 | 22±0.3 | 8.2±0.15** | 9.2±0.05** |
| | Group III | Calcium L-arabonate | 2.5 | 46±1.0** | 25±0.5** | 9.3±0.23** | 4.7±0.11** |
| | Group IV | Calcium D-gluconate | 2.5 | 46±1.0** | 29±0.5** | 8.6±0.21** | 4.4±0.08** |
| | Group V | Normal diet group | | 46±1.4 | 26±0.7 | 9.1±0.21 | 4.7±0.10 |

| | | | | | | | |
|---|---|---|---|---|---|---|---|
| Mean ± standard error; ++, p<0.01 vs Group V; *, p<0.05 vs Group I; **, p<0.01 vs Group I | | | | | | | |

The results of weight, ash content, calcium content and the like of femur and tibia are shown in Table 5. In the group for pathological condition control (Group I) which was subjected a combination of ovary excision and calcium-deficient diet, the weight, ash content, calcium content and phosphorus content of femur and tibia were all significantly reduced compared with those in the group excised ovaries but given normal feed (Group V), and their osteoporosis condition was more serious. The calcium L-arabonate administered groups (Groups II and III) were observed to be ameliorated in all of the indices in dose-dependent manner, and the efficacy was superior to those of the calcium D-gluconate administered group (Group IV).

### Example 9

### Preparation of food additive

The compositions (%) of the present invention (Example 9), Comparative Example 1 and Comparative Example 2 are shown in Table 6. The composition of the Comparative Example 2 was a commercially available food additive (pH adjusting agent).

**[Table 6]**

| Component | Example 9 | Comparative Example 1 | Comparative Example 2 |
|---|---|---|---|
| Sodium L-arabonate | 40.4 | - | - |
| L-arabono-γ-lactone | 30.0 | 48.0 | - |
| Trisodium citrate | 22.0 | 49.0 | - |
| Citric acid (anhydrate) | 4.6 | - | - |
| Tricalcium phosphate | 3.0 | 3.0 | - |
| Sodium malate | - | - | 55.4 |
| Malic acid | - | - | 12.0 |
| Phytic acid | - | - | 1.5 |
| Common salt | - | - | 31.5 |

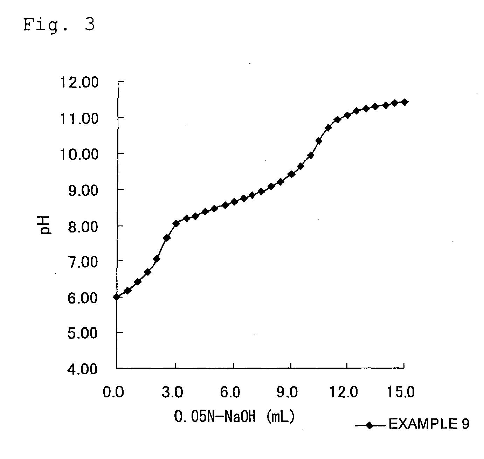

A sodium hydroxide solution at a concentration of 0.05 N was added to 50 mL of a 0.4% aqueous solution of the preparation of Example 9, and the changes in pH were obtained as shown in Fig. 3. As is obvious from Fig. 3, the preparation of Example 9 exhibited a mild and good buffering capacity. In addition, 0.2% aqueous solutions of the preparation of Example 9, Comparative Example 1 and Comparative Example 2 were tasted. The 0.2% aqueous solution of the preparation of Example 1 (pH 4.95), having the lowest pH among three, had the closest taste to water, no flavor, and thus it was indistinguishable from water. The preparations of Comparative Example 1 (pH 5.60) and Comparative Example 2 (pH 5.02) had clearly bitter tastes, even in small amount.

From these results, it was indicated that when the composition of the present invention was used as a food additive such as pH adjusting agent, the effects of pH adjustment could be utilized without affecting the original flavor and taste of the food.

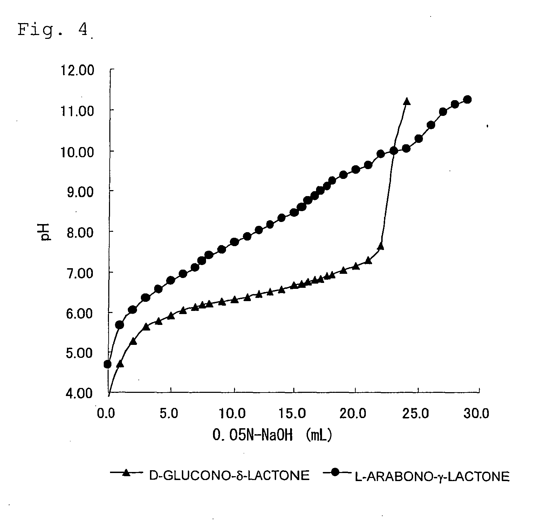

In addition, a sodium hydroxide solution at a concentration of 0.05 N was added to 50 mL of a 0.4% aqueous solution of L-arabono-γ-lactone and 50 mL of a 0.4% aqueous solution of D-glucono-δ-lactone, respectively, and the changes in pH were obtained as shown in Fig. 2. As is obvious from Fig. 4, the buffering capacity of L-arabono-γ-lactone was milder than that of D-glucono-δ-lactone at the same concentration, indicating that L-arabono-γ-lactone could be used as a food additive.

### Example 10

### Production of boiled noodles

Wheat flour (50 g of strong flour and 50 g of weak flour), 45 g of water, and 2 g of sodium L-arabonate were well kneaded and stood still for 20 minutes. The noodle dough was spread to a thickness of 2.5 mm, and was cut into noodles having a width of 2.2 mm to produce wheat noodles. The fresh noodles were boiled in boiling water for 7 minutes, and then were cooled with running water. Noodles produced by adding 2 g of common salt instead of sodium L-arabonate were also boiled in the same manner, and these noodles were compared. As a result, the noodles produced using sodium L-arabonate were indistinguishable from the noodles produced using common salt in the aspects of workability, cookability, appearance of the cooked noodles (color, flavor, surface of noodles), and texture upon eating. Thus, it was found that sodium L-arabonate could be used as a replacement of common salt, and that an effect of reducing salt ingestion could be obtained as well.

### Example 11

### Production of bread

For the production of bread, a domestic bread machine (Automatic Home Bakery HBS403, MK Seiko Co., Ltd.) was used. Raw materials for producing bread were 280 g of wheat flour (strong flour), 4 g of dry yeast, 30 g of white superior soft sugar, 5 g of skimmed milk, 10 g of butter, 190 g of water, and 4 g of sodium L-arabonate. Bread was also produced using 4 g of common salt instead of sodium L-arabonate, and these breads were compared. As a result, no difference was recognized between the bread using sodium L-arabonate and that using common salt in their workability. The bread produced using sodium L-arabonate did not have a salty taste, but no differences were recognized in their appearance, flavor and texture upon eating, as compared with the bread produced using common salt. Thus, it was found that sodium L-arabonate could be used as a replacement for common salt, and an effect of reducing salt ingestion could be obtained.

### Example 12

### Production of soybean curd

Soybean curd was produced from non-adjusted soymilk using a steamer. 2 g of L-arabono-γ-lactone was added to 500 mL of the cold non-adjusted soymilk (Meiraku Group), and slowly mixed. The mixture was transferred into a heatproof container, and the container was placed in a steamer in which steam was rising, with the lid off. The mixture was steamed at high heat for 10 minutes, and then at lower heat for 5 minutes. Then, the container was removed from the steamer, discarded the water accumulated on the surface of the mixture, and then cooled to get soybean curd. The texture of the soybean curd upon eating was very smooth, and no sour taste was occurred.

### Example 13

### Preparation of dressing

From all the compositions indicated in Table 7 excluding salad oil were thoroughly mixed with a whisk, and then salad oil was added in small portions to the mixture with rapidly stirring, to prepare a French dressing and a Japanese dressing.
As a result, for both of the French dressing and the Japanese dressing, the dressings with L-arabonic acid did not have the stimulating flavor characteristic of vinegar and had a mild taste, as compared with the dressings made from vinegar without L-arabonic acid.

**[Table 7]**

| | French dressing | | Japanese dressing | |
|---|---|---|---|---|
| | with L-arabonic acid | Conventional recipe (without L-arabonic acid) | with L-arabonic acid | Conventional recipe (without L-arabonic acid) |
| 4.2% aqueous L-arabonic acid solution | 25 mL | - | 50 mL | - |
| Vinegar (commercially available, 4.2%) | 25 mL | 50 mL | 50 mL | 100 mL |
| Salt | 3 g | 3 g | 5 g | 5 g |
| Pepper | 1 g | 1 g | 1 g | 1 g |
| Salad oil | 150 mL | 150 mL | 100 mL | 100 mL |
| Soy sauce | - | - | 60 mL | 60 mL |

### Example 14

### Preparation of beverage (sports drink)

The compositions indicated in Table 8 were dissolved in water to a volume of 1L to prepare sports drinks containing sodium L-arabonate and calcium L-arabonate.
As a result, the sports drinks containing sodium L-arabonate and calcium L-arabonate were clear without clouding, and having no differences in the taste from conventional sports drinks. It was found that since sodium L-arabonate and calcium L-arabonate do not cause cloudiness when dissolved in water and do not affect the flavor or taste of other compositions, the substances could be satisfactorily used in refreshing drinks and the like.

**[Table 8]**

| | Example 14 | Comp. Ex. |
|---|---|---|
| Sugar | 47 g | 47 g |
| Fructose | 11 g | 11 g |
| Glucose | 9 g | 9 g |
| Citric acid | 0.6 g | 0.6 g |
| Sodium L-arabonate | 0.5 g | - |
| Calcium L-arabonate | 0.15 g | - |
| Sodium citrate | - | 0.5 g |
| Calcium lactate | - | 0.15 g |
| Magnesium chloride | 0.05 g | 0.05 g |
| Sodium chloride | 0.76 g | 0.76 g |
| Potassium chloride | 0.4 g | 0.4 g |
| Lemon flavor | 1.5 g | 1.5 g |

### Example 15

Five grams of commercially available calcium L-arabonate (Sequoia Research Products, Ltd.) was dissolved in 500 mL of water to yield a solution containing L-arabonic acid. A glass column having an internal diameter of 2.0 cm and a length of 12 cm was charged with 20 mL of a cation exchange resin (Mitsubishi Chemical Corp., Diaion SK-1B), and the cation exchange resin was made into H type using 1 N hydrochloric acid. After washing the column with water, the solution containing L-arabonic acid was passed through the column to fractionate the solution into 50 mL each. The fractionated solution was analyzed by HPLC, and about 850 mL of the fraction containing L-arabonic acid was concentrated in an evaporator. The concentrated fraction in a syrup state was mixed with acetone with thorough stirring to homogenize. Furthermore, the concentration and addition of acetone were repeated, and the concentrated solution was crystallized at a low temperature (about 10°C). As a result, about 2.7 g of crystals were obtained.

The conditions for the HPLC analysis were as follows. Column: ICSepICE-ION-300 (Transgenomic, Inc.), 0.78 cm in internal diameter x 30 cm in length, column temperature: 70°C, eluent: 0.0085 N aqueous sulfuric acid solution, detector: differential refractometer detector (Showa Denko K. K., RI SE-61), flow rate: 0.4 mL/min, and retention time for eluted L-arabonic acid: approximately 17.5 min.

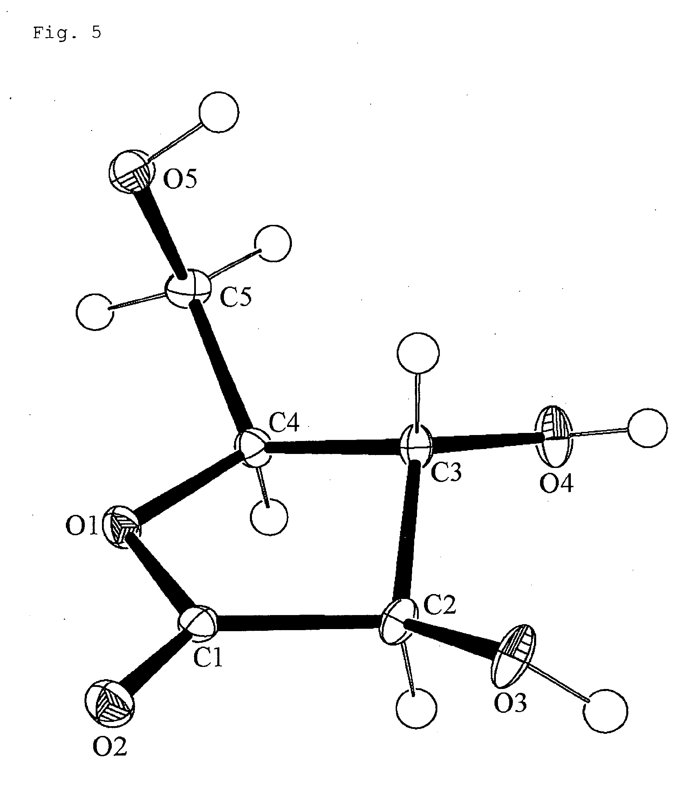

A portion of the obtained crystals was subjected to a structural analysis by X-ray crystal diffraction, and the crystals were confirmed to be of L-arabono-γ-lactone. The structure obtained by X-ray crystal diffraction was as presented in Fig. 5.

### Example 16

A 500-mL conical flask was filled with 100 mL of a culture medium (pH 7.0) containing 3.0% of ethanol, 0.15% of urea, 0.15% of ammonium sulfate, 0.15% of ammonium nitrate, 0.03% of yeast extract, 0.15% of KH₂PO₄, 0.15% of Na₂HPO₄·12H₂O, 0.03% of MgSO₄·7H₂O, 0.001% of CaCl₂·2H₂O, 0.001% of ZnSO₄·7H₂O, 0.001% of FeSO₄·7H₂O, and 0.0001% of MnSO₄·7H₂O. Acinetobacter baumannii was inoculated into the flask with one platinum loop. The flask was cultured with shaking at 30°C and 210 rpm for 2 days. One percent of the inoculation amount was inoculated into the similar culture medium, and the medium was cultured under the same conditions for 3 days. To the culture medium, a sterilized L-arabinose solutions (2 g/10 mL) was added to a final concentration of 2% and 0.5% (0.5 g) of dry-heat sterilized calcium carbonate was then added, and incubation was continued. After about 24 to 48 hours, L-arabinose was converted to L-arabonic acid.

A 60-fold volume of the culture medium in the above-mentioned conical flask (6 L) was centrifuged to prepare about 5.5L of the culture supernatant. Thirty grams of activated carbon was added to this supernatant, and the mixture was stirred at room temperature for 20 minutes and centrifuged. The resulting supernatant was filtered through a membrane filter (φ 0.45 µm). The filtrate of 5.3 L was concentrated in an evaporator. The concentration was stopped when an insoluble matter is appeared, and the filtrate was stood still at 4°C for one whole day. The resulting insoluble matter was collected by filtration to yield about 70 g of dry matter.
A glass column having an internal diameter of 4.5 cm and a length of 20 cm was charged with 270 mL of a cation exchange resin DOWEX 50 WX8 (The Dow Chemical Company), and the cation exchange resin was made into H type using 1 N hydrochloric acid and washed with water. The obtained dry matter in an amount of about 70 g was dissolved in about 5 L of distilled water, and the solution was passed through the column. The passed solution was fractionated into about 100 mL each, which was analyzed by HPLC in the same manner as in Example 15, and about 8 L of a fraction containing L-arabonic acid was obtained. About 8 L of the resulting L-arabonic acid solution was concentrated in an evaporator, and the concentrated fraction in a syrup state was mixed with acetone with thorough stirring to homogenize. Furthermore, the concentration and addition of acetone were repeated, and the concentrated solution was crystallized at a low temperature (about 10°C). As a result, about 41.65 g of crystals were obtained. The obtained crystals were confirmed to be of L-arabono-γ-lactone.

### Example 17

About 850 mL of an L-arabonic acid solution obtained in the same manner as in Example 15 was concentrated, and the concentrated solution in a syrup state was mixed with ethanol with thorough stirring to homogenize. Furthermore, the concentration and addition of ethanol were repeated, and the concentrated solution was crystallized at a low temperature (about 10°C). As a result, about 2.3 g of crystals were obtained. The obtained crystals were confirmed to be of L-arabono-γ-lactone.

### Example 18

From about 72 g of a dry matter obtained in the same manner as in Example 16, 10 g was dissolved in 500 mL of distilled water to yield a solution containing L-arabonic acid. A glass column having an internal diameter of 3.0 cm and a length of 15 cm was charged with 40 mL of a cation exchange resin DOWEX 50 WX8 (Dow Chemical Company). The cation exchange resin was made into H type using 1 N hydrochloric acid and was washed with water. Five-hundred milliliters of the L-arabonic acid solution was passed through the column. The passed solution was fractionated into about 50 mL each, which was analyzed by HPLC in the same manner as in Example 15, and about 950 mL of the resulting L-arabonic acid solution was concentrated in an evaporator. The concentrated solution in a syrup state is mixed with acetonitrile with thorough stirring to homogenize. Furthermore, the concentration and addition of acetonitrile were repeated, and a small amount of the crystals obtained in Example 16 was added thereto as seed crystals to perform crystallization from the concentrated solution at a low temperature (about 10°C). As a result, about 4.9 g of crystals were obtained. The obtained crystals were confirmed to be of L-arabono-γ-lactone.

### Example 19

From 41.65 g of the crystals obtained in Example 16, about 10 g was dissolved in a mixed solvent of acetone and ethanol (1:1). The solution was concentrated, and then crystallization was performed from the concentrated solution at a low temperature (about 10°C). As a result, about 6.1 g of crystals were obtained. The obtained crystals were confirmed to be of L-arabono-γ-lactone.

### Example 20

From 41.65 g of the crystals obtained in Example 16, about 10 g was dissolved in acetone. The solution was concentrated in an evaporator, and then ethyl acetate was added thereto in small portions to perform crystallization at a low temperature (about 10°C). As a result, about 6.3 g of crystals were obtained. The obtained crystals were confirmed to be of L-arabono-γ-lactone.

### Example 21

From 41.65 g of the crystals obtained in Example 16, about 10 g was dissolved in a mixed solvent of acetone and methanol (4:1). The solution was concentrated in an evaporator. Then, ethyl acetate was added thereto in small portions, and a small amount of the crystals obtained in Example 16 was added as seed crystals, to perform crystallization at a low temperature (about 10°C). As a result, about 6.4 g of crystals were obtained. The obtained crystals were confirmed to be of L-arabono-γ-lactone.

### Industrial Applicability

The poorly absorbable L-form aldonic acid, a salt or lactone thereof disclosed in the present invention has a prominent action of accelerating calcium absorption in the small intestine, which is a predominant absorption site for calcium. Ingestion of a food product to which the composition disclosed in the invention has been previously added, or ingestion of the composition of the invention together with calcium-containing food products such as dairy products, fish or meat, improves the absorption rate of calcium which is contained in the food products but poorly absorbed. Consequently, the decreased bone mineral content in osteoporosis is increased, thus the risk for bone fracture being decreased.

## Claims

1. A composition for accelerating calcium absorption, comprising a poorly absorbable L-form aldonic acid, a salt thereof, or a poorly absorbable L-form aldonolactone.

2. A composition for prevention and treatment of osteoporosis, comprising a poorly absorbable L-form aldonic acid, a salt thereof, or a poorly absorbable L-form aldonolactone.

3. The composition according to claim 1 or 2, wherein the poorly absorbable L-form aldonic acid is L-arabonic acid.

4. The composition according to any one of claims 1 to 3, which is a pharmaceutical composition or a composition for food product.

5. A calcium supplement food product containing calcium L-arabonate.

6. A dietary product containing L-arabonic acid, a salt thereof, or L-arabonolactone.

7. A food additive containing L-arabonic acid, a salt thereof, or L-arabonolactone.

8. The food additive according to claim 7, which is used in dietary products, wherein the dietary products are noodles, pickles, articles of taste, prepared foods, vegetables, cooked rice, processed rice cake products, frozen fish, processed fish products, processed meat products, fries, soybean paste, sauces, fruit juice beverages, refreshing drinks, or dairy products.

9. A process for preparing L-arabono-γ-lactone crystals, **characterized by** dissolving L-arabonic acid in an organic solvent, and crystallizing L-arabono-γ-lactone from the resulting solution.

10. A process for preparing L-arabono-γ-lactone crystals, **characterized by** crystallizing L-arabonic acid from acetone, methanol, ethanol, acetonitrile, or a mixed solvent thereof.

11. A process for preparing L-arabono-γ-lactone crystals, **characterized by** dissolving L-arabonic acid in acetone, methanol, ethanol, acetonitrile, or a mixed solvent thereof, and then adding ethyl acetate or propanol thereto.

12. The process according to any one of claims 9 to 11, wherein the L-arabonic acid is obtained by making a microorganism or enzyme which is capable of aldose oxidation act on L-arabinose.
